# NEW EUROPEAN PATENT SPECIFICATION

(11) **EP 2 173 413 B2**
(45) Date of publication and mention of the opposition decision: **07.08.2019**
(45) Mention of the grant of the patent: 30.03.2011
(21) Application number: 08776083.1
(22) Date of filing: 28.07.2008
(51) Int. Cl.: A61M 5/20

(54) **INJECTION DEVICE**
INJEKTIONSVORRICHTUNG
DISPOSITIF D'INJECTION

(30) Priority: 08.08.2007 GB 0715461
(43) Date of publication of application: 14.04.2010
(73) Proprietor: Cilag GmbH International, 6300 Zug (CH)
(72) Inventor: JENNINGS, Douglas Ivan, Royston, Hertfordshire SG8 7XU (GB)
(74) Representative: Tunstall, Christopher Stephen
(86) International application number: PCT/GB2008/002579
(87) International publication number: WO 2009/019438

(56) References cited:
- WO-A-03/013632
- WO-A-2007/036676
- WO-A1-99/37343
- WO-A1-03/097133
- WO-A1-2005/115510
- WO-A1-2005/115516
- WO-A2-92/19296
- WO-A2-2004/054645
- WO-A2-2006/062788
- US-A- 3 320 955
- US-A- 5 681 291
- US-A1- 2004 225 262
- US-A1- 2005 203 466

## Description

### Field of the Invention

The present invention relates to an injection device of the type which has a syringe and which extends the syringe, discharges its contents and then retracts it automatically.

### Background of the Invention

Injection devices are shown in WO 95/35126 and EP-A-0 516 473. These devices employ a drive spring and some form of release mechanism that releases the syringe from the influence of the drive spring once its contents are supposed to have been discharged, to allow it to be retracted by a return spring.

Generally, the return spring is relatively weak, since its restoring force must be overcome by the drive spring, even while the drive spring is doing work on the various components of the injection device and the syringe during an injection cycle. This may give rise to a problem when the injection device is used with sealed hypodermic syringes, which typically have a hermetically sealed cover, needle shield or "boot" that covers the hypodermic needle and maintains the sterility of the syringe contents. Naturally, it is necessary to maintain the sterility of the syringe contents up to the point of administration, which for devices that are designed to be disposable, as many will be, means that the boot must be removed with the syringe inside the injection device.

Typically, the action required to remove the boot from the syringe is simply to pull the boot away from the syringe, which requires a force in excess of 20N. This is significantly greater than the restoring force of the return spring, so the syringe will be pulled out of the injection device as the boot is removed and, when the boot comes away, it will snap back into place. This is not the best way to handle the syringe. The shock could damage it, the needle could be damaged and there may be problems re-engaging the syringe with those components of the injection device designed to act upon it. Even in cases where there is no return spring, for example where the syringe is held in place by friction with components of the injection device, the problem will still arise of relocating the syringe onto those components of the injection device designed to act upon it.

Moreover, there is a problem with having the syringe generally moveable in a direction out of the injection device. Accidental activation of the drive spring by mechanical failure of the drive spring's release mechanism (e.g. a trigger) can occur, for example by dropping the device on a hard surface. This accidental activation could cause the syringe to be extended unintentionally out of the device and its contents to be ejected. This could expose the needle of the syringe and increase the risk of inadvertent ski puncturing and/or infection.

US 2005/203466 A1 discloses an injection device comprising a locking mechanism including latching sleeve and latching tongues. The locking mechanism of this document is provided between a syringe carrier and the housing of the injection device.

### Summary of the Invention

The injection device of the present invention is designed to deal with the aforementioned problems.

The present invention, provides an injection device according to claim 1.

Thus, the syringe carrier and syringe cannot move towards the exit aperture prior to activation of the injection device. Since the locking mechanism is built into the drive, there is no requirement for a separate, independent locking mechanism. The syringe carrier becomes unlocked automatically (i.e. movement towards the exit aperture is permitted) when then drive is moved to cause the syringe and syringe carrier to move into the extended position. By preventing movement of the syringe carrier out of the exit aperture (unless this is during an actuation sequence of the injection device), damage to the syringe and its contents is prevented. Moreover, this assists in preventing accidental activation of the injection device, for example by dropping the injection device on a hard surface.

Preferably the injection device comprises a release mechanism adapted, in an engaged position, to prevent the actuator acting on the drive and, in a disengaged position, to permit the actuator to act on the drive, wherein the locking mechanism is adapted to prevent movement of the syringe carrier towards the exit aperture when the release mechanism is in its engaged position.

Preferably, the locking mechanism comprises at least one arm fixed relative the syringe carrier, wherein the arm is engageable with a corresponding locking surface on the drive.

In one embodiment of the present invention, the locking mechanism comprises a plurality of arms on the syringe carrier spaced circumferentially around the drive. This provides enhanced locking against movement of the syringe towards the exit aperture.

Advantageously, the arms may be spaced equidistantly around the drive.

In a particular embodiment of the present invention, the locking mechanism comprises two arms.

Preferably, each arm is flexible and/or resilient. This permits the drive to be inserted into the syringe through the end of the syringe carrier because the arms flex outwards. More preferably, each arm protrudes in a direction towards the longitudinal axis.

Preferably, each arm is positioned and directed to contact the locking surface on the drive to prevent movement of the syringe carrier in the longitudinal direction towards the exit aperture.

The injection device has a proximal end adjacent the exit aperture and a distal end located at an opposite end of the injection device along the longitudinal axis.

In one embodiment of the present invention, the locking surface is formed between a first section of the drive and a second section of the drive, wherein the second section of the drive is located towards the distal end of the drive with respect to the first section, wherein the locking surface is formed on the intersection between the first and second sections of the drive.

Preferably, the first section of the drive is positioned nearer to the exit aperture than the at least one arm. The second section of the drive may be narrower about the longitudinal axis than the first section of the drive.

Preferably, the locking surface is formed by a surface which is not parallel to the longitudinal direction.

More preferably, the locking surface is formed by a surface which is perpendicular to the longitudinal direction.

In a particular embodiment of the present invention, the drive comprises a shaft which extends along the longitudinal axis.

Preferably, the actuator comprises biasing means adapted to bias the syringe carrier from a retracted position to an extended position.

This arrangement of the drive and arms permits the wider proximal end of the drive to pass through the arms until such point that a narrower section of the drive is located adjacent the arms. At this point, the arms flex back into their normal unbiased position, thereby preventing the wider proximal end of the drive from passing back through the syringe carrier and out of the syringe. This also means that the syringe carrier cannot move towards the exit aperture unless it moves in conjunction with the drive.

In one embodiment of the present invention, the drive includes first and second drive elements, of which the first is acted upon by the actuator and in turn acts upon the second, and the second acts upon the syringe or the syringe carrier to advance it from its retracted position to its extended position and discharge its contents through the discharge nozzle, the first drive element being capable of movement relative to the second when the first is acted upon by the actuator and the second is restrained by the syringe or the syringe carrier.

Preferably, the injection device comprises a coupling that prevents the first drive element from moving relative to the second until they have been advanced to a nominal decoupling position that is less advanced than the said nominal release position.

Advantageously, the coupling may comprise a decoupling mechanism, activated when the drive elements have been advanced to the said nominal decoupling position and adapted to decouple the first drive element from the second, thus allowing the first drive element to move relative to the second.

In one embodiment of the present invention, the injection device further comprises a cap removably located over the exit aperture.

Preferably, the injection device compres a needle shield removably located over the discharge nozzle.

The cap may be adapted to grip the needle shield, such that the needle shield is released and removed from the discharge nozzle during removal of the cap from the housing.

### Brief Description of the Drawings

The invention will now be described by way of example with reference to the accompanying drawings, in which:
Fig. 1a is a right-side view of the injection device according to the present invention;
Fig. 1b is a perspective view of the injection device of Fig. 1 with its cap removed;
Fig. 1c is a perspective view of the cap of the injection device of Fig. 1;
Fig. 2a is an exploded right-side view of the injection device of Fig. 1;
Fig: 2b is a right-side view of the assembled components of the injection device of Fig. 1;
Fig. 2c is a perspective view of a multi-component drive used in the injection device of Fig. 1
Fig. 3 is a cross-sectional view of the injection device of Fig. 1.

### Detailed Description of the Drawings

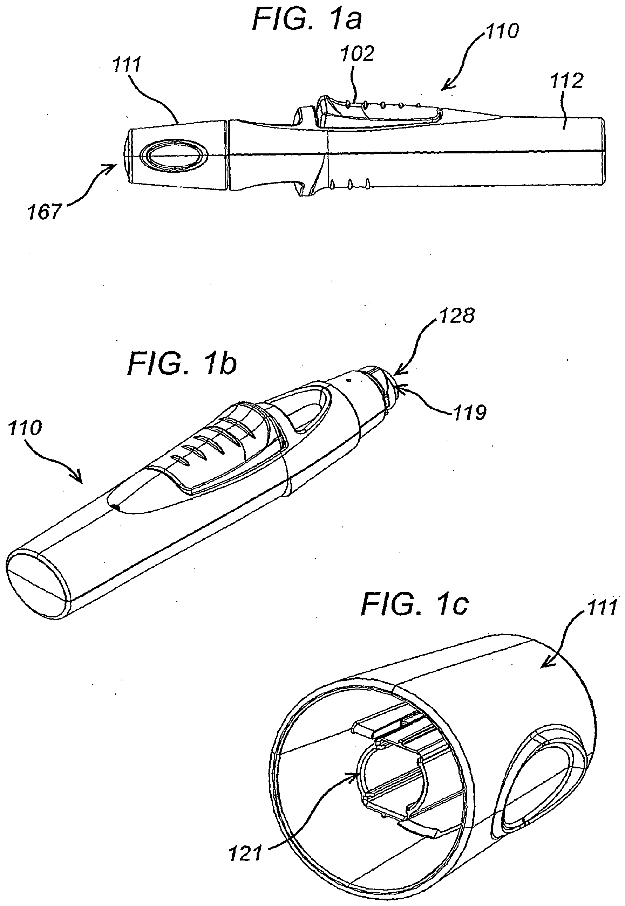

Fig. 1a is a right-side view of an injection device 110 according to the present invention. The injection device 110 has a housing 112, a cap 111 which is removable from a proximal end 167 the housing 112 and a trigger button 102. Other parts of the device will be described in greater detail below.

Fig. 1b is a perspective view of the injection device 110 according to the present invention with the cap (not shown) removed from its end. The end of the housing 112 has an exit aperture 128, from which the end of a sleeve 119 can be seen to emerge.

Fig. 1c is a perspective view of the cap 111 of the injection device 110 according to the present invention. The cap 111 has a central boss 121 that fits within the sleeve 119 when the cap 111 is installed on the housing 112.

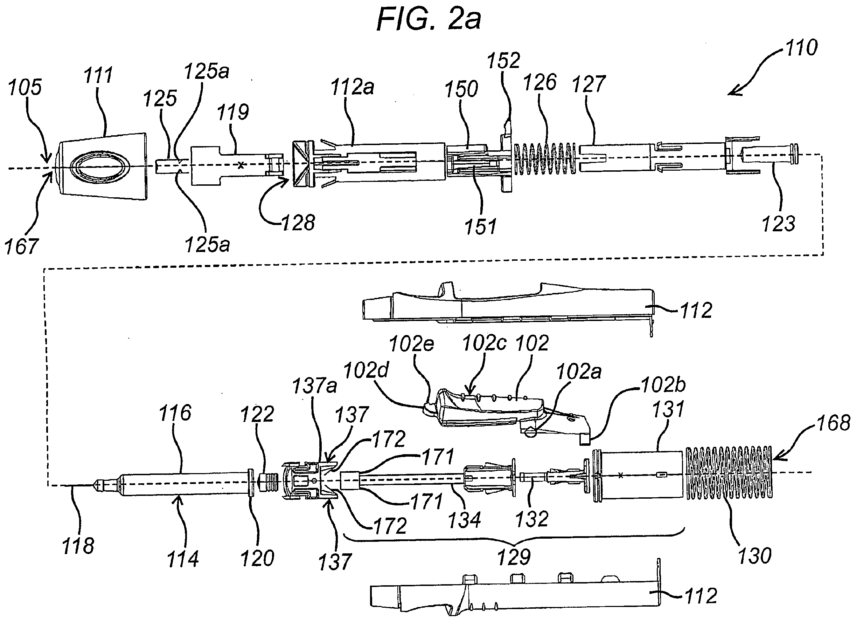

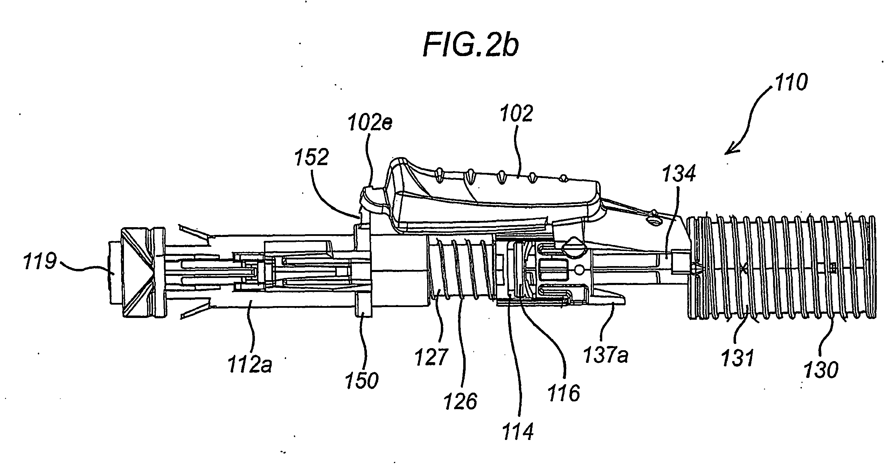

Fig. 2a is an exploded right-side view of the components of the injection device 110 according to the present invention and Fig. 2b is a right-side view of the assembled components of the injection device 110 according to the present invention without the housing 112 or cap 111.

As illustrated, the injection device 110 comprises a hypodermic syringe 114 of conventional type, including a syringe body 116 terminating at one end in a discharge nozzle, specifically a hypodermic needle 118, and at the other in a flange 120. The conventional plunger that would normally be used to discharge the contents of the syringe 114 manually has been removed and replaced with a drive element (referred to below as the second drive element 134) that contacts a bung 122 in the syringe 114. The bung 122 constrains a drug (not shown) to be administered within the syringe body 116. Whilst the syringe illustrated is of hypodermic type, this need not necessarily be so. Transcutaneous or ballistic dermal and subcutaneous syringes may also be used with the injection device of the present invention.

As illustrated, the injection device 110 includes a return spring 126 that biases the syringe 114 from an extended position in which the needle 118 extends from the aperture 128 in a case nose 112a of the housing 112 to a retracted position in which the needle 118 is contained within the housing 112. The return spring 126 acts on the syringe 114 via a syringe carrier 127. The syringe 114 is moveable along a longitudinal axis 105 of the injection device 110 which extends centrally along the length of the injection device 110 from the exit aperture 128 at its proximal end 167 to a distal end 168.

Contained within the housing at its distal end 168 is an actuator, which here takes the form of a compression drive spring 130. Drive from the drive spring 130 is transmitted via a multi-component drive 129 to the syringe 114 to advance it from its retracted position to its extended position and discharge its contents through the needle 118. The drive 129 accomplishes this task by acting directly on the drug and the syringe 114. Hydrostatic forces acting through the drug and, to a lesser extent, static friction between the bung 122 and the syringe body 116 initially ensure that they advance together, until the return spring 126 bottoms out on the syringe carrier 127 or meets some other obstruction (not shown) that retards its motion.

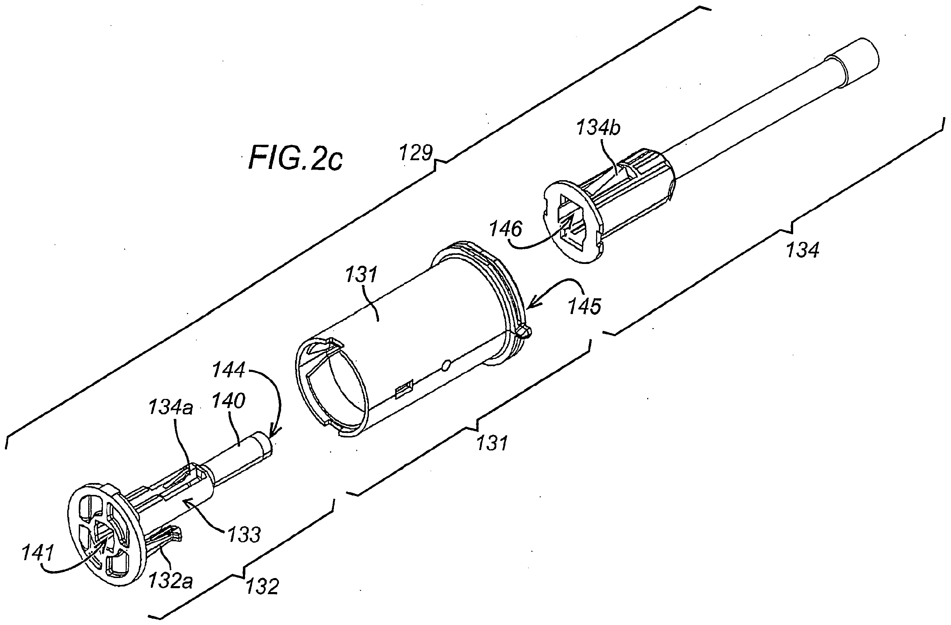

Fig. 2c is an exploded perspective view of the multi-component drive 129. The multi-component drive 129 between the drive spring 130 and the syringe 114 consists of three principal components. A drive sleeve 131 takes drive from the drive spring 130 and transmits it to a delay piston 133 on a first drive element 132. This in turn transmits drive to the second drive element 134.

As will be seen from Fig. 2c, the first drive element 132 includes a hollow stem 140, the inner cavity of which forms a collection chamber 141 in communication with a vent 144 that extends from the collection chamber 141 through the end of the stem 140. The second drive element 134 includes a blind bore 146 that is open at one end to receive the stem 140 and closed at the other. As will be appreciated, the bore 146 and the stem 140 define a fluid reservoir within which a damping fluid is contained.

The trigger button 102 is provided on the side of the housing 112 which, when in an engaged position with a proximal end 145 of the drive sleeve 131, serves to retain the drive spring 130 in a compressed state by contact between locking surface 102b and the drive sleeve 131 when the trigger button 102 is in an unactuated position. The trigger button 102 can pivot on the housing 112 via pivot 102a. When downwards pressure is applied to the trigger button 102 at an activation surface 102c (i.e. pressure directed into the housing 112), the locking surface 102b moves upwards in a direction away from the longitudinal axis 105. In this actuated position of the trigger button 102, the locking surface 102b is decoupled from the drive sleeve 131, thereby allowing the drive sleeve 131 to move relative to the housing 112 towards the exit aperture 128 under the influence of the drive spring 130.

The sliding sleeve 119 is moveable from its extended position (as shown in Fig. 1b) where it protrudes out of the exit aperture 128 into a retracted position in the case nose 112a of the housing 112. The sliding sleeve 119 is connected to a trigger button lock element 150 which has resilient arms 151 which bias the sliding sleeve 119 into its extended position in which its end protrudes from the end of the case nose 112a. Thus, application of pressure to the end of the sliding sleeve 119, for example by pressing the end of the sliding sleeve 119 against tissue, causes it to move into its retracted position into the housing 112; release of the pressure causes the sliding sleeve 119 to move into its extended position under bias from the resilient arms 151 acting against a side wall of the housing 112. The trigger button lock element 150 has a trigger button lock protrusion 152 which contacts with the end of a trigger button protrusion 102d on the trigger button 102 when the sliding sleeve is in its extended position. The trigger button protrusion 102d extends in a direction which is generally parallel to the longitudinal axis 105 of the injection device 110. The trigger button lock protrusion 152 extends in a direction which is generally perpendicular to the longitudinal axis 105 towards the trigger button protrusion 102d. The trigger button protrusion 102d has an aperture 102e which can move over the top of the trigger button lock protrusion 152 when the trigger button lock element 150 has been moved away from the exit aperture 128 (i.e. when the sliding sleeve 119 has been moved into the exit aperture 128 into its retracted position). In this position, the trigger button 102 can be moved into its deactivated position by rotating the trigger button 102 about the pivot 102a in the direction of the pressure applied to the pressure surface 102c. Thus, the trigger button lock element 150 and the sliding sleeve 119 act together to lock the trigger button 102 in its activated position (i.e. the locking surface 102b contacts the end of the drive sleeve 131 preventing it from moving towards the exit aperture 128 under the bias of the compressed drive spring 130).

When the sliding sleeve 119 has been moved into a position in which it is retracted into the housing 112 (i.e. into its unlocked position) and the trigger button 102 has been rotated into its deactivated position, the operation of the device 110 is then as follows.

Initially, the drive spring 130 moves the drive sleeve 131, the drive sleeve 131 moves the first drive element 132 and the first drive element 132 moves the second drive element 134, in each case by acting through flexible latch arms 132a, 134a, 134b. The second drive element 134 moves and, by virtue of static friction and hydrostatic forces acting through the drug (not shown), moves the syringe body 116 and syringe carrier 127 against the action of the return spring 126. The return spring 126 compresses and the hypodermic needle 118 emerges from the exit aperture 128 of the housing 112. This continues until the return spring 126 bottoms out or the syringe body 116 meets some other obstruction (not shown) that retards its motion. Because the static friction between the second drive element 134 and the syringe body 116 and the hydrostatic forces acting through the drug (not shown) to be administered are not sufficient to resist the full drive force developed by the drive spring 130, at this point the second drive element 134 begins to move within the syringe body 116 and the drug (not shown) begins to be discharged. Dynamic friction between the second drive element 134 and the syringe body 116 and hydrostatic forces acting through the drug (not shown) to be administered are, however, sufficient to retain the return spring 126 in its compressed state, so the hypodermic needle 118 remains extended.

Before the second drive element 134 reaches the end of its travel within the syringe body 116, so before the contents of the syringe have fully discharged, the flexible latch arms 134a, 134b linking the first and second drive elements 132, 134 reach a constriction 137 provided on a latch actuator element 137a which is fixed to the end of the syringe carrier 127. The constriction 137 moves the flexible latch arms 134a, 134b inwards from the position shown in Fig. 2c to a position at which the flexible latch arms 134a, 134b no longer couple the first drive element 132 to the second drive element 134, aided by the bevelled surfaces on the constriction 137. Once this happens, the first drive element 132 acts no longer on the second drive element 134, allowing the first drive element 132 to move relative to the second drive element 134.

Because the damping fluid is contained within a reservoir (not shown) defined between the end of the first drive element 132 and the blind bore 146 in the second drive element 134, the volume of the reservoir will tend to decrease as the first drive element 132 moves relative to the second drive element 134 when the former is acted upon by the drive spring 130. As the reservoir collapses, damping fluid is forced through the vent 144 into the collection chamber 141. Thus, once the flexible latch arms 134a, 134b have been released, the force exerted by the drive spring 130 does work on the damping fluid, causing it to flow though the constriction formed by the vent 144, and also acts hydrostatically through the fluid and through friction between the first and second drive elements 132, 134, thence via the second drive element 134. Losses associated with the flow of the damping fluid do not attenuate the force acting on the body of the syringe to a great extent. Thus, the return spring 126 remains compressed and the hypodermic needle remains extended.

After a time, the second drive element 134 completes its travel within the syringe body 116 and can go no further. At this point, the contents of the syringe 114 are completely discharged and the force exerted by the drive spring 130 acts to retain the second drive element 134 in its terminal position and to continue to cause the damping fluid to flow though the vent 144, allowing the first drive element 132 to continue its movement.

Before the reservoir of fluid is exhausted, the flexible latch arms 132a linking the drive sleeve 131 with the first drive element 132 reach another constriction (not shown) within the housing 112. This constriction moves the flexible latch arms 132a inwards from the position shown to a position at which they no longer couple the drive sleeve 131 to the first drive element 132, aided by bevelled surfaces on the constriction. Once this happens, the drive sleeve 131 acts no longer on the first drive element 132, allowing them to move relative each other. At this point, of course, the syringe 1 14 is released, because the forces developed by the drive spring 130 are no longer being transmitted to the syringe 114, and the only force acting on the syringe will be the return force from the return spring 126. Thus, the syringe 114 is now returned to its retracted position and the injection cycle is complete.

All this takes place, of course, only once the cap 111 has been removed from the end of the housing 112. The end of the syringe is sealed with a boot 123. The central boss 121 of the cap that fits within the sleeve 119 when the cap 111 is installed on the housing 112 comprises a retainer element 125 which is fixed into the boss 121. The retainer element 125 comprises resilient protrusions 125a which are directed away from the exit aperture 128. These resilient protrusions 125a deform as the cap 111 is inserted onto the housing 112 over a needle shield or rubber boot 123. The protrusions 125a then grip the boot 123 tightly so that the ends of the protrusions are slightly embedded in the boot 123 which might be made from rubber. This means that, as the cap 111 is pulled off the housing 112, the boot 123 is pulled away from the syringe 114 with the cap 111.

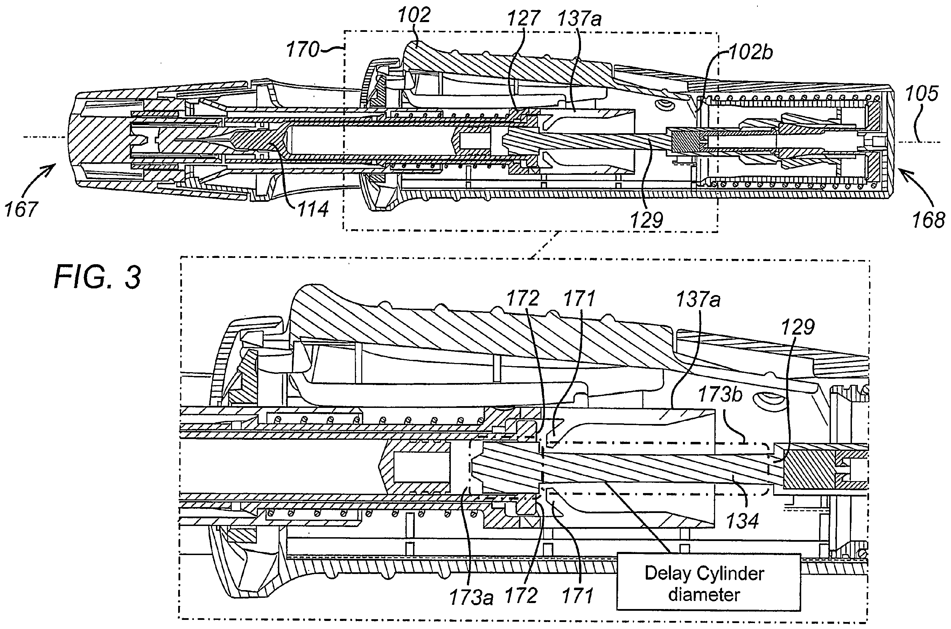

Fig. 3 shows how the locking mechanism 170 is integrated with the injection device 110 of the present invention.

The locking mechanism 170 is located on the latch actuator element 137a so that it is always located between the syringe carrier 127 and the drive 129 to inhibit movement of the syringe carrier 127 and syringe 114 towards the proximal end 167 of the injection device unless the drive 129 itself moves in that direction.

The locking mechanism comprises a plurality of arms 171 that are fixed to the latch actuator element 137a so that they are fixed relative the syringe carrier 127. Each arm is engageable with a corresponding locking surface 172 on the drive 129. The arms 171 are spaced circumferentially and equidistantly about the longitudinal axis 105.

Each arm 171 is flexible thereby permitting the drive 129 to be inserted into the distal end of the syringe 114 during assembly of the device. Each arm 171 protrudes in a direction towards the longitudinal axis 105 and along it (i.e. at an oblique angle to the longitudinal axis 105) towards the proximal end 167 of the injection device 110. This means that the arms 171 are positioned and directed to contact the locking surface 172 on the drive 129 to prevent movement of the syringe carrier 127 and syringe 114 towards the exit aperture in a direction parallel to the longitudinal axis 105.

The locking surface 172 is formed between a first section 173a of the drive and a second section 173b of the drive 129, specifically on the second drive element 134 . The second section 173b of the drive 129 is located towards the distal end 168 of the injection device 110 with respect to the first section 173a. The locking surface 172 is formed on the surface which joins the first and second sections 173a, 173b of the drive 129. The first section 173a of the drive is positioned nearer to the proximal end 167 of the injection device 110 than the arms 171. The second section 173b of the drive is narrower about the longitudinal axis 105 than the first section 173a of the drive, thereby forming a ridge for the locking surface 172 in the drive 129.

When an attempt is made to move the syringe carrier 127 and syringe 114 in a direction towards the proximal end 167 of the injection device 110 (when such movement is not via the drive 129, for example when removing the cap 111 by pulling it in a direction away from the proximal end 167 of the housing 112), the arms 171 come into contact with the locking surface 172, but cannot pass the locking surface 172. This means that the force pulling the syringe 114 in a direction towards the proximal end 167 is transmitted via the drive 129 to the locking surface 102b on the button 102. This prevents the syringe 114 and syringe carrier 127 from moving longitudinally unless caused to do so through movement of the drive 129.

It will of course be understood that the present invention has been described above purely by way of example and modifications of detail can be made within the scope of the invention.

## Claims

1. An injection device (110) comprising:
a housing (112) adapted to receive a syringe (114) having a discharge nozzle (118), the syringe (114) being moveable in the housing (112) along a longitudinal axis from a retracted position in which the discharge nozzle (118) is contained within the housing (112) and an extended position in which the discharge nozzle (118) of the syringe (114) extends from the housing (112) through an exit aperture (128);
an actuator (130);
a drive (129) adapted to be acted upon by the actuator (130) and in turn act upon the syringe (114) to advance it from its retracted position to its extended position and discharge its contents through the discharge nozzle (118);
a syringe carrier (127) adapted to support the syringe (114) as it is advanced;
a return spring that biases the syringe (114) from the extended position to the retracted position, wherein the return spring acts on the syringe via the syringe carrier; and
a locking mechanism (170) between the syringe carrier (127) and the drive (129) to inhibit movement of the syringe carrier (127) and syringe (114) towards the exit aperture (128).

2. The injection device (110) according to claim 1, further comprising a release mechanism adapted, in an engaged position, to prevent the actuator (130) acting on the drive (129) and, in a disengaged position, to permit the actuator (130) to act on the drive (129), wherein the locking mechanism (170) is adapted to prevent movement of the syringe carrier (127) towards the exit aperture (128) when the release mechanism is in its engaged position.

3. The injection device (110) of claim 1 or claim 2, wherein the locking mechanism (170) comprises at least one arm fixed relative the syringe carrier (127), wherein the arm is engageable with a corresponding locking surface on the drive (129).

4. The injection device (110) of claim 3, wherein the locking mechanism (170) comprises a plurality of arms on the syringe carrier (127) spaced circumferentially around the drive (129).

5. The injection device (110) of claim 4, wherein the arms are spaced equidistantly around the drive (129).

6. The injection device (110) of claim 4 or claim 5, wherein the locking mechanism (170) comprises two arms.

7. The injection device (110) of any one of claims 3 to 6, wherein each arm is flexible.

8. The injection device (110) of any one of claims 3 to 6, wherein each arm protrudes in a direction towards the longitudinal axis.

9. The injection device (110) of any one of claims 3 to 6, wherein each arm is positioned and directed to contact the locking surface on the drive (129) to prevent movement of the syringe carrier (127) in the longitudinal direction towards the exit aperture (128).

10. The injection device (110) of any one of claims 3 to 9, wherein the injection device (110) has a proximal end adjacent the exit aperture (128) and a distal end located at an opposite end of the injection device (110) along the longitudinal axis.

11. The injection device (110) of claim 10, wherein the locking surface is formed between a first section of the drive (129) and a second section of the drive (129), wherein the second section of the drive (129) is located towards the distal end of the drive (129) with respect to the first section,
wherein the locking surface is formed on the intersection between the first and second sections of the drive (129).

12. The injection device (110) of claim 11, wherein the first section of the drive (129) is positioned nearer to the exit aperture (128) than the at least one arm.

13. The injection device (110) of any one of claims 11 or 12, wherein the second section of the drive (129) is narrower about the longitudinal axis than the first section of the drive (129).

14. The injection device (110) of any one of claims 11 to 13, wherein the locking surface is formed by a surface which is not parallel to the longitudinal direction.

15. The injection device (110) of claim 14, wherein the locking surface is formed by a surface which is perpendicular to the longitudinal direction.

16. The injection device (110) of claim 15, wherein the drive (129) comprises a shaft which extends along the longitudinal axis.

17. The injection device (110) of claim 16, wherein the actuator (130) comprises biasing means adapted to bias the syringe carrier (127) from a retracted position to an extended position.

18. The injection device (110) of any one of the preceding claims, wherein the drive (129) includes first and second drive elements, of which the first is acted upon by the actuator (130) and in turn acts upon the second, and the second acts upon the syringe (114) or the syringe carrier (127) to advance it from its retracted position to its extended position and discharge its contents through the discharge nozzle (118), the first drive element being capable of movement relative to the second when the first is acted upon by the actuator (130) and the second is restrained by the syringe (114) or the syringe carrier (127).

19. The injection device (110) of any one of the preceding claims, further comprising a coupling that prevents the first drive element from moving relative to the second until they have been advanced to a nominal decoupling position that is less advanced than the said nominal release position.

20. The injection device (110) of any one of the preceding claims, wherein the coupling comprises a decoupling mechanism, activated when the drive elements have been advanced to the said nominal decoupling position and adapted to decouple the first drive element from the second, thus allowing the first drive element to move relative to the second.

21. The injection device (110) of any one of the preceding claims, further comprising a cap (111) removably located over the exit aperture (128).

22. The injection device (110) of any one of the preceding claims, further comprising a needle shield (123) removably located over the discharge nozzle (118).

23. The injection device (110) of claim 22 when dependent on claim 21, wherein the cap (111) is adapted to grip the needle shield (123), such that the needle shield (123) is released and removed from the discharge nozzle (118) during removal of the cap (111) from the housing (112).

## Patentansprüche

1. Injektionsvorrichtung (110), welche Folgendes umfasst:
ein Gehäuse (112), welches eingerichtet ist, um eine **Spritze** (114) mit einer Auslassdüse (118) aufzunehmen, wobei die Spritze (114) in dem Gehäuse (112) entlang einer Längsachse aus einer zurückgezogenen Position, in welcher die Auslassdüse (118) innerhalb des Gehäuses (112) enthalten ist, in eine ausgefahrene Position, in welcher sich die Auslassdüse (118) der Spritze (114) aus dem Gehäuse (112) durch eine Austrittsöffnung (128) erstreckt, beweglich ist,
ein **Betätigungselement** (130);
einen **Antrieb** (129), welcher eingerichtet ist, um durch das Betätigungselement (130) betätigt zu werden und wiederum die Spritze (114) zu betätigen, so dass sie von ihrer zurückgezogenen Position in ihre ausgefahrene Position vorbewegt wird und ihren Inhalt durch die Auslassdüse (118) auslässt;
einen Spritzenträger (127), welcher eingerichtet ist, um die Spritze (114) zu stützen, während sie vorbewegt wird;
einen Blockiermechanismus (170) zwischen dem Spritzenträger (127) und dem Antrieb (129), um eine Bewegung des Spritzenträgers (127) und der Spritze (114) **in** Richtung zu der Austrittsöffnung (128) hin zu hemmen.

2. Injektionsvorrichtung (110) nach Anspruch 1, welche weiter einen Freigabemechanismus umfasst, welcher eingerichtet ist, um in einer eingekoppelten Position zu verhindern, dass das Betätigungselement (130) den Antrieb (129) betätigt, und um es in einer ausgekoppelten Position dem Betätigungselement (130) zu ermöglichen, den Antrieb (129) zu betätigen, wobei der Blockiermechanismus (170) eingerichtet ist, um eine Bewegung des Spritzenträgers (127) in Richtung der Austrittsöffnung (128) zu verhindern, wenn sich der Freigabemechanismus in seiner eingekoppelten Position befindet.

3. Injektionsvorrichtung (110) nach Anspruch 1 oder Anspruch 2, wobei der Blockiermechanismus (170) zumindest einen Arm umfasst, welcher relativ zu dem Spritzenträger (127) fixiert ist, wobei der Arm in eine entsprechende Blockierfläche auf dem Antrieb (129) einkoppelbar ist.

4. Injektionsvorrichtung (110) nach Anspruch 3, wobei der Blockiermechanismus (170) mehrere Arme auf dem Spritzenträger (127) umfasst, welche peripher um den Antrieb (129) herum beabstandet sind.

5. Injektionsvorrichtung (110) nach Anspruch 4, wobei die Arme in gleichem Abstand um den Antrieb (129) herum angeordnet sind.

6. Injektionsvorrichtung (110) nach Anspruch 4 oder Anspruch 5, wobei der Blockiermechanismus (170) zwei Arme umfasst.

7. Injektionsvorrichtung (110) nach einem der Ansprüche 3 bis 6, wobei jeder Arm flexibel ist.

8. Injektionsvorrichtung (110) nach einem der Ansprüche 3 bis 6, wobei jeder Arm in eine Richtung zu der Längsachse hin vorsteht.

9. Injektionsvorrichtung (110) nach einem der Ansprüche 3 bis 6, wobei jeder Arm positioniert und ausgerichtet ist, um die Blockieroberfläche auf dem Antrieb (129) zu berühren, um eine Bewegung des Spritzenträgers (129) in Längsrichtung zu der Austrittsöffnung (128) hin zu verhindern.

10. Injektionsvorrichtung (110) nach einem der Ansprüche 3 bis 9, wobei die Injektionsvorrichtung (110) ein proximales Ende neben der Austrittsöffnung (128) und ein distales Ende, welches sich an einem entlang der Längsachse entgegengesetzten Ende der Injektionsvorrichtung (110) befindet, aufweist.

11. Injektionsvorrichtung (110) nach Anspruch 10, wobei die Blockieroberfläche zwischen einem ersten Abschnitt des Antriebs (129) und einem zweiten Abschnitt des Antriebs (129) gebildet ist, wobei sich der zweite Abschnitt des Antriebs (129) bezüglich des ersten Abschnitts in Richtung auf das distale Ende des Antriebs (129) hin befindet, wobei die Blockieroberfläche an der Überschneidung zwischen dem ersten und dem zweiten Abschnitt des Antriebs (129) gebildet ist.

12. Injektionsvorrichtung (110) nach Anspruch 11, wobei der erste Abschnitt des Antriebs (129) näher an der Austrittsöffnung (128) positioniert ist als der zumindest eine Arm.

13. Injektionsvorrichtung (110) nach einem der Ansprüche 11 oder 12, wobei der zweite Abschnitt des Antriebs (129) enger um die Längsachse herum angeordnet ist als der erste Abschnitt des Antriebs (129).

14. Injektionsvorrichtung (110) nach einem der Ansprüche 11 bis 13, wobei die Blockieroberfläche durch eine Oberfläche gebildet ist, welche nicht parallel zu der Längsrichtung ist.

15. Injektionsvorrichtung (110) nach Anspruch 14, wobei die Blockieroberfläche durch eine Oberfläche gebildet ist, welche senkrecht zu der Längsrichtung ist.

16. Injektionsvorrichtung (110) nach Anspruch 15, wobei der Antrieb (129) einen Schaft umfasst, welcher sich entlang der Längsachse erstreckt.

17. Injektionsvorrichtung (110) nach Anspruch 16, wobei das Betätigungselement (130) ein Vorspannmittel umfasst, welches eingerichtet ist, um den Spritzenträger (127) aus einer zurückgezogenen Position in eine ausgefahrene Position vorzuspannen.

18. Injektionsvorrichtung (110) nach einem der vorhergehenden Ansprüche, wobei der Antrieb (129) ein erstes und ein zweites Antriebselement aufweist, wovon das erste durch das Betätigungselement (130) betätigt wird und wiederum das zweite betätigt, und wobei das zweite die Spritze (114) oder den Spritzenträger (127) betätigt, so dass sie aus ihrer zurückgezogenen Position in ihre ausgefahrene Position vorbewegt wird und ihren Inhalt durch die Auslassdüse (118) auslässt, wobei das erste Antriebselement zu einer Bewegung relativ zu dem zweiten in der Lage ist, wenn das erste von dem Betätigungselement (130) betätigt wird und das zweite durch die Spritze (114) oder den Spritzenträger (127) zurückgehalten wird.

19. Injektionsvorrichtung (110) nach einem der vorhergehenden Ansprüche, welche weiter eine Kopplung umfasst, welche verhindert, dass sich das erste Antriebselement relativ zu dem zweiten bewegt, bis sie in eine nominelle Entkopplungsposition vorbewegt sind, welche weniger vorbewegt ist als die nominelle Freigabeposition.

20. Injektionsvorrichtung (110) nach einem der vorhergehenden Ansprüche, wobei die Kopplung einen Entkopplungsmechanismus umfasst, welcher aktiviert ist, wenn die Antriebselemente zu der nominellen Entkopplungsposition vorbewegt sind, und welcher eingerichtet ist, um das erste Antriebselement von dem zweiten zu entkoppeln, wodurch dem ersten Antriebselement ermöglicht ist, sich relativ zu dem zweiten zu bewegen.

21. Injektionsvorrichtung (110) nach einem der vorhergehenden Ansprüche, welche weiter eine Kappe (111) umfasst, welche sich abnehmbar über der Austrittsöffnung (128) befindet.

22. Injektionsvorrichtung (110) nach einem der vorhergehenden Ansprüche, welche weiter eine Nadelabschirmung (123) umfasst, welche sich abnehmbar über der Auslassdüse (118) befindet.

23. Injektionsvorrichtung (110) nach Anspruch 22, wenn dieser von Anspruch 21 abhängt, wobei die Kappe (111) eingerichtet ist, um die Nadelabschirmung (123) zu fassen, so dass die Nadelabschirmung (123) während einer Abnahme der Kappe (111) von dem Gehäuse (112) freigegeben und von der Auslassdüse (118) abgenommen wird.

## Revendications

1. Dispositif d'injection (110) comprenant :
un logement (112) adapté pour recevoir une seringue (114) qui présente une buse de décharge (118), la seringue (114) étant mobile dans le logement (112) le long d'un axe longitudinal à partir d'une position rétractée dans laquelle la buse de décharge (118) est contenue à l'intérieur du logement (112), et une position déployée dans laquelle la buse de décharge (118) de la seringue (114) se déploie à partir du logement (112) à travers une ouverture de sortie (128) ;
un actionneur (130) ;
un entraînement (129) adapté pour être actionné par l'actionneur (130) et qui agit à son tour sur la seringue (114) de façon à la faire avancer à partir de sa position rétractée vers sa position déployée et à décharger son contenu à travers la buse de décharge (118);
un support de seringue (127) adapté pour supporter la seringue (114) à mesure qu'elle est avancée;
un ressort de rappel qui sollicite la seringue (114) à partir de la position déployée vers la position rétractée, dans lequel le ressort de rappel agit sur la seringue par le biais du support de seringue ; et
un mécanisme de verrouillage (170) entre le support de seringue (127) et l'entraînement (129) de façon à empêcher un déplacement du support de seringue (127) et de la seringue (114) vers l'ouverture de sortie (128).

2. Dispositif d'injection (110) selon la revendication 1, comprenant en outre un mécanisme de libération adapté, dans une position en prise, pour empêcher l'actionneur (130) d'agir sur l'entraînement (129) et, dans une position désengagée, pour permettre à l'actionneur (130) d'agir sur l'entraînement (129), dans lequel le dispositif de verrouillage (170) est adapté pour empêcher un déplacement du support de seringue (127) vers l'ouverture de sortie (128) lorsque le mécanisme de libération se trouve dans sa position en prise.

3. Dispositif d'injection (110) selon la revendication 1 ou la revendication 2, dans lequel le mécanisme de verrouillage (170) comprend au moins un bras fixe par rapport au support de seringue (127), dans lequel le bras peut venir en prise avec une surface de verrouillage correspondante sur l'entraînement (129).

4. Dispositif d'injection (110) selon la revendication 3, dans lequel le mécanisme de verrouillage (170) comprend une pluralité de bras situés sur le support de seringue (127) et espacés de manière circonférentielle autour de l'entraînement (129).

5. Dispositif d'injection (110) selon la revendication 4, dans lequel les bras sont espacés de manière équidistante autour de l'entraînement (129).

6. Dispositif d'injection (110) selon la revendication 4 ou la revendication 5, dans lequel le mécanisme de verrouillage (170) comprend deux bras.

7. Dispositif d'injection (110) selon l'une quelconque des revendications 3 à 6, dans lequel chaque bras est flexible.

8. Dispositif d'injection (110) selon l'une quelconque des revendications 3 à 6, dans lequel chaque bras fait saillie dans une direction vers l'axe longitudinal.

9. Dispositif d'injection (110) selon l'une quelconque des revendications 3 à 6, dans lequel chaque bras est positionné et dirigé de façon à entrer en contact avec la surface de verrouillage sur l'entraînement (129) de manière à empêcher un déplacement du support de seringue (127) dans la direction longitudinale vers l'ouverture de sortie (128).

10. Dispositif d'injection (110) selon l'une quelconque des revendications 3 à 9, dans lequel le dispositif d'injection (110) présente une extrémité proximale adjacente à l'ouverture de sortie (128) et une extrémité distale située à une extrémité opposée du dispositif d'injection (110) le long de l'axe longitudinal.

11. Dispositif d'injection (110) selon la revendication 10, dans lequel la surface de verrouillage est formée entre une première section de l'entraînement (129) et une seconde section de l'entraînement (129), dans lequel la seconde section de l'entraînement (129) se situe vers l'extrémité distale de l'entraînement (129) par rapport à la première section ;
dans lequel la surface de verrouillage est formée sur l'intersection entre les première et seconde sections de l'entraînement (129).

12. Dispositif d'injection (110) selon la revendication 11, dans lequel la première section de l'entraînement (129) est positionnée plus près de l'ouverture de sortie (128) que le ou les bras.

13. Dispositif d'injection (110) selon l'une quelconque des revendications 11 ou 12, dans lequel la seconde section de l'entraînement (129) est plus étroite autour de l'axe longitudinal que la première section de l'entraînement (129).

14. Dispositif d'injection (110) selon l'une quelconque des revendications 11 à 13, dans lequel la surface de verrouillage est formée par une surface qui n'est pas parallèle à la direction longitudinale.

15. Dispositif d'injection (110) selon la revendication 14, dans lequel la surface de verrouillage est formée par une surface qui est perpendiculaire à la direction longitudinale.

16. Dispositif d'injection (110) selon la revendication 15, dans lequel l'entraînement (129) comprend une tige qui s'étend le long de l'axe longitudinal.

17. Dispositif d'injection (110) selon la revendication 16, dans lequel l'actionneur (130) comprend des moyens de sollicitation adaptés pour solliciter le support de seringue (127) à partir d'une position rétractée vers une position déployée.

18. Dispositif d'injection (110) selon l'une quelconque des revendications précédentes, dans lequel l'entraînement (129) comprend des premier et second éléments d'entraînement, dont le premier est actionné par l'actionneur (130) et agit à son tour sur le second, et le second agit sur la seringue (114) ou sur le support de seringue (127) de façon à le faire avancer à partir de sa position rétractée vers sa position déployée et à décharger son contenu à travers la buse de décharge (118), le premier élément d'entraînement étant capable de se déplacer par rapport au second lorsque le premier est actionné par l'actionneur (130) et le second est retenu par la seringue (114) ou par le support de seringue (127) .

19. Dispositif d'injection (110) selon l'une quelconque des revendications précédentes, comprenant en outre un accouplement qui empêche le premier élément d'entraînement de se déplacer par rapport au second jusqu'à ce qu'ils aient été avancés vers une position de désaccouplement nominale qui est moins avancée que ladite position de libération nominale.

20. Dispositif d'injection (110) selon l'une quelconque des revendications précédentes, dans lequel l'accouplement comprend un mécanisme de désaccouplement, activé lorsque les éléments d'entraînement ont été avancés vers ladite position de désaccouplement nominale et adapté pour désaccoupler le premier élément d'entraînement du second, en permettant de ce fait au premier élément d'entraînement de se déplacer par rapport au second.

21. Dispositif d'injection (110) selon l'une quelconque des revendications précédentes, comprenant en outre une coiffe (111) située de manière amovible sur l'ouverture de sortie (128).

22. Dispositif d'injection (110) selon l'une quelconque des revendications précédentes, comprenant en outre un dispositif de protection d'aiguille (123) situé de manière amovible sur la buse de décharge (118).

23. Dispositif d'injection (110) selon la revendication 22 lorsqu'elle dépend de la revendication 21, dans lequel la coiffe (111) est adaptée pour saisir le dispositif de protection d'aiguille (123), de telle sorte que le dispositif de protection d'aiguille (123) soit libéré et retiré de la buse de décharge (118) au cours du retrait de la coiffe (111) du logement (112).
